# EUROPÄISCHE PATENTSCHRIFT

(11) **EP 2 496 201 B1**
(45) Veröffentlichungstag und Bekanntmachung des Hinweises auf die Patenterteilung: **02.01.2019**
(21) Anmeldenummer: 10795608.8
(22) Anmeldetag: 01.11.2010
(51) Int. Cl.: A61K 6/00

(54) **HAFTVERMITTLER ZWISCHEN OXIDKERAMIK UND EINEM VERBLENDWERKSTOFF, INSBESONDERE FÜR DENTALE ZWECKE, VERFAHREN ZU SEINER ANWENDUNG SOWIE KIT ZU SEINER HERSTELLUNG UND APPLIKATION**
ADHESION PROMOTER BETWEEN OXIDE CERAMIC AND A VENEER MATERIAL, IN PARTICULAR FOR DENTAL PURPOSES, METHOD FOR THE USE THEREOF AND KIT FOR THE PRODUCTION AND APPLICATION THEREOF
PROMOTEUR D'ADHÉSION ENTRE UNE CÉRAMIQUE D'OXYDE ET UN MATÉRIAU DE REVÊTEMENT, EN PARTICULIER À USAGE DENTAIRE, SON PROCÉDÉ D'UTILISATION ET KIT POUR SA PRODUCTION ET SON APPLICATION

(30) Priorität: 02.11.2009 DE 102009051593
(43) Veröffentlichungstag der Anmeldung: 12.09.2012
(73) Patentinhaber: Xplus3 GmbH, 89284 Pfaffenhofen (DE)
(72) Erfinder: GÖBEL, Roland, 07749 Jena (DE); GLÜCK, Olaf, 61209 Echzell (DE)
(74) Vertreter: Donath, Dirk
(86) Internationale Anmeldenummer: PCT/DE2010/001262
(87) Internationale Veröffentlichungsnummer: WO 2011/050786

(56) Entgegenhaltungen:
- DD-A1- 276 453
- DE-A1-102005 042 091
- DATABASE WPI Week 200856 Thomson Scientific, London, GB; AN 2008-J50842 & CN 101 058 698 A (CHANGGE TAIPING SANITARY CERAMIC FACTORY) 24 October 2007 (2007-10-24)

## Beschreibung

Die Erfindung betrifft einen Haftvermittler zwischen einer synthetisch hergestellten oder aus Naturmineralien gemischten Oxidkeramik, bestehend aus einem oder mehreren Metalloxiden wie insbesondere Zirkoniumoxid-, Aluminiumoxid- oder Spinell-Keramik, sowie einem auf diese aufzubringenden Verblendwerkstoff, beispielsweise Silikatkeramik, Verblendkomposite oder Verblendkunststoff, insbesondere für dentale Zwecke.
Die Erfindung umfasst auch ein Verfahren zur Anwendung des Haftvermittlers, um einen solchen Werkstoffverbund herzustellen, sowie einen Kit zur Herstellung und Applikation des Haftvermittlers.
Oxidkeramiken werden bekannter Weise zur Herstellung hoch belastbarer dentaler Kronen und Brücken verwendet, wobei es erforderlich ist, zur ästhetischen Gestaltung solcher prothetischen Gerüste die relativ opake Oxidkeramikoberfläche mit einer zahnfarbenen Dentalkeramik, nachfolgend Verblendkeramik genannt (in Form von Silikatkeramik, oder auch als Feldspatkeramik oder Glaskeramik bezeichnet, hergestellt aus den Hauptbestandteilen Feldspart und Quarz) zu versehen. Analog kann die Verblendung mit zahnfarbenem Verblendkomposit oder Verblendkunststoff erfolgen. Dabei wird das Gerüst zunächst mit diamantierten Instrumenten aus einem vorgesinterten Oxidkeramik-Block herausgefräst. Dieses Teil ist vom Volumen etwa 20 % größer als das spätere zu verblendende Gerüst. Im anschließenden Sinterbrand (1250 °C bis 1600 °C) schrumpft das Gerüst auf die eigentliche Passform. Auf dieses vorgefertigte und nun dicht gesinterte Oxidkeramikteil wird die Verblendkeramik aufgebracht und ebenfalls gesintert (850 °C bis 1000 °C). Dabei wird angestrebt, dass die Ausdehnungskoeffizienten beider Keramiken möglichst identisch sind. Aus technologischer Sicht ist der Ausdehnungskoeffizient der Verblendkeramik geringfügig kleiner als der der Gerüstkeramik, so dass beim Aufsintern und dem danach erfolgten Abkühlungsprozess die Verblendkeramik auf die Oxidkeramik im mechanischen Verbund aufgeschrumpft wird (z. B. Eichner, Kappert: Zahnärztliche Werkstoffe und ihre Verarbeitung, Band 2, Werkstoffe und ihre klinische Verarbeitung, Thieme Verlag).

Unter den extremen Bedingungen des Mundmilieus, der stetigen Feuchte- und Temperaturwechsel sowie mechanischen Belastungen treten gerade an der Grenzfläche beider Keramiken extreme Spannungen auf, die dazu führen, dass die Verblendkeramik direkt von der Oberfläche der Oxidkeramik abplatzen kann bzw. dass die Spannungen in die Verblendkeramik weitergeleitet werden, so dass innere Spannungen in der Verblendkeramik auftreten, die zu Kohäsionsbrüchen und damit zu Abplatzungen in der Keramik führen ("chipping").

Aufgrund dieser zahlreichen Probleme versuchte die Fachwelt, die Verbundfestigkeit durch eine Silikatschicht auf der Oxidkeramik zu erhöhen, wobei mehrere Methoden zu deren Aufbringung offenbart wurden.
In der US 4,364,731 A ist ein Verfahren beschrieben, mit dem eine Siliziumdioxidschicht mittels Hochfrequenz-Magnetron-Sputter-Vorrichtung aufgebracht wird.
Bekannt ist auch (DE 34 03 894 C1), eine Silikatschicht durch einen Flammenhydrolyseprozess von Tetraethoxysilan aufzubringen.
Weiterhin ist in DD 276 453 ein Verfahren beschrieben, bei dem eine Silikat-Chromoxid-Schicht durch eine Sol-Gel-Lösung aufgebracht und durch einen nachfolgenden Temperprozess (320 °C, 2-8 min) verfestigt wird.
In der DE 38 02 043 C1 wird eine Methode gezeigt, bei der die Silikatbeschichtung durch einen Korundstrahlprozess erfolgt, wobei dem Strahlkorund eine gewisse Menge Siliziumdioxid einer mittleren Partikelgröße < 5 µm zugesetzt wird. Im Aufschlagbereich der Korundteilchen treten dabei örtlich Energiedichten auf, die ausreichend sind, die feinteiligen Silikatpartikel auf die Oberfläche aufzuschmelzen. Allen vorgenannten Bemühungen ist gemein, dass sie einen aufwendigen und teuren apparativen und verfahrenstechnischen Aufwand erfordern, aber keinen wirklichen Qualitätssprung zur Erhöhung der Verbundfestigkeit und zur Unterdrückung des besagten "chippings" bewirken.

Die DE 10 2005 042 091 A1 offenbart einen keramischen Zahnersatz, insbesondere keramische Verbundkronen oder keramische Verbundbrücken, bestehend aus zwei unabhängigen Bestandteilen, welche in einem computergestützten Verfahren hergestellt werden und durch eine keramische Konnektormasse miteinander verbunden sind, sowie auf ein Verfahren zu dessen Herstellung. Der keramische Zahnersatz wird im CAD/CAM-Verfahren aus zwei Einzelkomponenten hergestellt, welche in einem Fügeschritt miteinander verbunden werden. Die innere Gerüststruktur besteht dabei entweder aus einem Metallgerüst oder aus einer hochfesten Gerüstkeramik, die äußere Verblendkappe wird aus einem silikatischen Verblendkeramikmaterial gefräst. Der Verbund beider Strukturen erfolgt mittels einer niedriger schmelzenden keramischen Masse in einem Sinterprozess.

Aus der DD 276 453 A1 ist eine Haftvermittler- Schicht zwischen einer Dentallegierung und einer Verblendung aus Kunststoff bekannt, welche aus Siliziumdioxid und einem oder mehreren Metalloxiden besteht und die einen Konzentrationsgradienten aufweißt, wobei die Metalloxidkonzentration in der Schicht in Richtung auf den Kunststoff hin abnimmt. Als Dispergator des Haftvermittlers wird Wasser und Isopropanol oder Aceton und Isopropanol verwendet, um eine solartige Konsistenz des Haftvermittlers zu erzielen.
Die DD 276 453 A offenbart darüber hinaus ein Verfahren zur Herstellung eines dentalen Metall/Kunststoff- Verbundkörpers, bei dem eine kolloidale Dispersion von Siliziumdioxid als Haftvermittler auf die zu verblendende Oberfläche aufgetragen wird und durch einen nachfolgenden Temperprozess bei 100 bis 800°C (insbesondere bei 380°C) verfestigt wird.

Der Erfindung liegt die Aufgabe zugrunde, den Verbund zwischen der Oxidkeramik und dem Verblendwerkstoff zu verbessern sowie dessen Haltbarkeit zu erhöhen.

Erfindungsgemäß wird der Verblendwerkstoff nicht unmittelbar auf den vorgefertigten Grundkörper aus dicht gesinterter Oxidkeramik aufgebracht, sondern es wird zunächst ein passfähiger Grundkörper aus noch nicht dicht gesinterter Oxidkeramik oder deren Ausgangsmaterialien hergestellt. Aus Gründen der Fertigung kann der Grundkörper zwar bei niedrigeren Temperaturen vorgesintert, nicht jedoch, wie bisher, dicht gesintert sein. Auf die zu verblendende Oberfläche dieses so vorgefertigten Grundkörpers wird nunmehr ein Haftvermittler als Sol (Schlicker) aufgetragen, wobei dieser in die Oberfläche des noch nicht dicht gesinterten Grundkörpers (je nach Verwendung bis zu 10 µm Tiefe) eindringt.
Der Haftvermittler besteht aus einem Gemisch aus Feldspat- und Quarzpartikeln im Mischungsverhältnis zwischen 95 : 05 und 10 : 90, sowie aus einem geeigneten Dispergator (Dispergiermittel), beispielsweise Wasser.

Nach Aufbringung des Haftvermittlers wird die behandelte Oberfläche des Grundkörpers getrocknet (beispielsweise durch Umgebungsluft oder Heizung) bzw. gehärtet oder polymerisiert.
Erst danach wird der Grundkörper bei Temperaturen von 1250 °C bis 1600 °C dicht gesintert, wodurch die Oxidkeramik letztendlich ihre volle mechanische Festigkeit und Robustheit erreicht.
Im Anschluss daran wird der Verblendwerkstoff (beispielsweise Silikatkeramik oder Verblendkunststoff) in an sich bekannter Weise auf den nunmehr dicht gesinterten Grundkörper, in dessen Oberfläche vorher erfindungsgemäß der besagte Haftvermittler einwirkte, aufgebracht.

Durch den besagten Sinterbrand der Oxidkeramik werden die eindiffundierten Feldspat- und Quarzpartikel in das Oxidkeramikgerüst eingebunden. Für dentale Anwendungen wird eine beispielsweise als Verblendwerkstoff aufgebrachte Verblendkeramik (Feldspatkeramik) ebenfalls gesintert (bei Temperaturen von 850 °C bis 1.000 °C), wobei einerseits die Keramikstrukturen der Verblendkeramik ausgebildet werden; andererseits kommt es, was für den Verbund von entscheidender Bedeutung ist, zu Metall-Sauerstoff-Silizium-Bindungen zwischen den fest verankerten Metalloxiden in der Oxidkeramikmatrix und dem Silikat in der Feldspatkeramik bzw. dem Quarz. Diese Reaktion garantiert ähnlich dem klinisch über Jahrzehnte erprobten Verbund zwischen den Dentallegierungen und Verblendkeramik einen zusätzlichen optimalen chemischen Verbund zwischen Oxid- und Verblendkeramik.

Überraschend hat sich gezeigt, dass durch den in die Oberfläche der noch nicht dicht gesinterten Oxidkeramik eingedrungenen Haftvermittler nicht nur ein festerer und damit höher beanspruchbarer beständigerer Verbund an der Grenzfläche zwischen dem Grundkörper und dem Verblendwerkstoff geschaffen wird, sondern eigene Untersuchungen haben zusätzlich ergeben, dass auch die Gefahr des so genannten "chipping" (kohäsivem Abplatzen oder Ausbrechen von Bereichen des Verblendwerkstoffes in sich) verringert ist, was auf veränderte mechanische Spannungsverhältnisse im Bereich der besagten Grenzfläche zurückgeführt wird, die auf das Eindiffundieren des Haftvermittlers in die Oberfläche der noch nicht dicht gesinterten Oxidkeramik zurückzuführen sind. Ursächlich hierfür dürfte sein, dass mit der Erfindung nicht nur, wie bisher ein mechanischer Verbund zwischen der Oxidkeramik und dem Verblendwerkstoff geschaffen wird, sondern dass der Haftvermittler vor dem endgültigen Sintern der Oxidkeramik in deren vorschlagsgemäß behandelte Oberfläche bis zu 10 µm in die Oberfläche eindiffundiert und auf diese Weise beim Aufbringen des Verblendwerkstoffes die Voraussetzungen für eine zusätzliche chemische Bindung schafft. Durch das Endsintern der Oxidkeramik werden die eindiffundierten Silikatstrukturen fest in die Oxidkeramik eingebunden. Ganz entscheidend ist dieses Einbinden allerdings an der Oberfläche, da mit dem anschließenden Aufbringen der Verblendkeramik ebenfalls eine Silikatkeramik aufgebracht wird, die beim Sintern chemisch über Si-O-Si - Bindungen mit dem oberflächennah eingebundenen Silikat reagiert und damit ein chemischer Verbund zwischen Oxid- und Verblendkeramik gebildet wird.
Die bekannten Verfahren zur Erhöhung der Verbundfestigkeit konnten keine solche chemische Bindung ermöglichen und waren deshalb auf die rein mechanische Verbundfestigkeit des beschriebenen Aufschrumpfens beschränkt. Der verbesserte Verbund zwischen der Oxidkeramik und dem Verblendwerkstoff wirkt nicht nur bei der Verwendung von Verblendkeramik (Silikatkeramik), sondern auch für andere Verblendmaterialien, wie insbesondere Verblendkomposite oder Kunststoffe.

Es ist möglich, Haftvermittler der beschriebenen Art zur fertigen Sol-Anwendung bereitzustellen. Andererseits könnte es zweckmäßig sein, den Haftvermittler anwendungsspezifisch speziell herzustellen. Hierfür wäre ein Kit dienlich, mit welchem die Ausgangsmaterialien und ggf. Utensilien und Instrumente zur Applikation bereitgestellt werden. Ein solcher Kit könnte beispielsweise zumindest bestehen aus:
- wenigstens einem Behältnis, enthaltend einen Verblendwerkstoff, wie Silikatkeramik, beispielsweise Feldspat,
- wenigstens einem Behältnis mit Quarz,
- wenigstens einem Behältnis mit einem Dispergator, beispielsweise Wasser,
- Instrumente zum Anmischen des Haftvermittlers und/oder dessen Auftragen sowie
- einer Vorschrift zur Handhabung des Kits (Herstellung und/oder Applikation des Haftvermittlers.

Die Erfindung soll nachstehend an zwei Ausführungsbeispielen näher erläutert werden.

### Beispiel 1:

Auf einen bei Temperaturen im Bereich zwischen 600 °C und 900 °C vorgesinterten Oxidkeramikkörper (95 % ZrO₂, 5 % Y₂O₃) mit den Abmaßen 2 x 15 x 15 mm wird ein Sol, bestehend aus 7,5 g Feldspat, 2,5 g Quarz und 100 ml destilliertem Wasser, aufgetragen. Die Auftragung erfolgt mittels eines weichen Pinsels.

Das Sol (Wasser mit den Feststoffpartikeln) diffundiert in den oberflächennahen Bereich der Oxidkeramik ein. Die Feldspat- und Quarzpartikel verbleiben dort, während das Wasser nach 1 Minute verdunstet ist. Nach dem Einwirken und Trocknen des Sols erfolgt bei ca. 1600 °C das Endsintern des Oxidkeramikkörpers. Anschließend wird die Verblendkeramik (Zirox) in einer Schichtstärke von 1 mm aufgetragen und wie verfahrensüblich bei einer Temperatur von 930 °C gesintert. Um die Festigkeit des Werkstoffverbundes Oxidkeramik - Verblendkeramik messen zu können, wird auf die Verblendkeramik zu den besagten Testzwecken ein Kunststoffzylinder ( 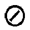 = 5 mm, h = 2 mm) aufmodelliert. Auf diesen Kunststoffzylinder wird mittels Druck-Scher-Belastung eine Kraft ausgeübt, die bei entsprechend hoher Belastung dazu führt, dass der Kunststoffzylinder mit der Verblendkeramik von der Oxidkeramik abgelöst wird, allerdings so, dass der Bruch stets in der Verblendkeramik erfolgt. Ein Teil der Verblendkeramik verbleibt auf der Oxidkeramik, der andere Teil auf dem abgescherten Kunststoffzylinder. Der Mittelwert der gemessenen Verbundwerte betrug 25 MPa. Wird der gleiche Versuch an endgesinterten Oxidkeramikkörpern (ohne SolAuftrag) durchgeführt, zeigt sich im Bruchverhalten stets, dass die Verblendkeramik komplett von der Oxidkeramik entfernt ist und sich vollständig auf dem abgescherten Kunststoffzylinder befindet. Der Mittelwert der gemessenen Verbundwerte betrug in diesen Vergleichsfällen 20 MPa.

### Beispiel 2:

Auf einen bei Temperaturen im Bereich zwischen 600 °C und 1.000 °C vorgesinterten Oxidkeramikkörper (95 % ZrO₂ , 5 % Y₂O₃) mit den Abmaßen 2 x 15 x 15 mm wird ein Sol, bestehend aus 5 g Feldspat, 5 g Quarz und 100 ml destilliertem Wasser, aufgetragen. Die Auftragung erfolgt mittels eines weichen Pinsels.

Das Sol (Wasser mit den Feststoffpartikeln) diffundiert in den oberflächennahen Bereich der Oxidkeramik ein. Die Feldspat und Quarzpartikel verbleiben dort, während das Wasser nach 1 Minute verdunstet ist. Nach dem Einwirken und Trocknen des Sols erfolgt bei ca. 1.450 °C das Endsintern des Oxidkeramikkörpers. Auf diesen Oxidkeramikkörper wird ein methacrylathaltiges Haftsilan (Siliseal) aufgetragen. Dieses Silan wird verwendet, um bei Feldspatabplatzungen diese mit Kunststoff zu reparieren. Mittels dieses Silans wird ein optimaler chemischer Feldspatkeramik-Kunststoff-Verbund ermöglicht. Anschließend wird ein Kunststoffzylinder (Verblendkunststoff: Sinfony, Ø = 5 mm, h = 2 mm) aufmodelliert. Auf diesen Kunststoffzylinder wird mittels Druck-Scher-Belastung eine Kraft ausgeübt, die bei entsprechend hoher Belastung dazu führt, dass der Kunststoffzylinder von der Oxidkeramik abgelöst wird, allerdings so, dass der Bruch stets im Verblendkunststoff erfolgt. Der Mittelwert der gemessenen Verbundwerte betrug 20 MPa. Wird der gleiche Versuch an endgesinterten Oxidkeramikkörpern (ohne Sol-Auftrag) durchgeführt, zeigte sich stets ein adhäsives Bruchverhalten an der Oxidkeramikoberfläche. Der Mittelwert der gemessenen Verbundwerte betrug in diesen Vergleichsfällen 8 MPa.

Durch den erfindungsgemäßen Vorschlag wird zusätzlich eine chemische Anbindung von Oxid- und Verblendkeramik bzw. Oxidkeramik und Verblendkunststoff erreicht. Bei Belastung erfolgt der Bruch stets im schwächsten Glied (in diesem Fall ein Kohäsionsbruch in der Verblendkeramik bzw. ein Kohäsionsbruch im Verblendkunststoff), während ohne erfindungsgemäße Vorgehensweise das schwächste Glied der Verbundkombination die Grenzfläche Oxidkeramik-Verblendkeramik ist, wobei ein adhäsives Bruchverhalten gegeben ist.

## Patentansprüche

1. Verfahren zur Anwendung eines Haftvermittlers für die Herstellung eines Verbundes zwischen einer Oxidkeramik, bestehend aus einem oder mehreren Metalloxiden sowie einem auf die Oxidkeramik aufzubringenden Verblendwerkstoff, bestehend aus Silikatkeramik, Verblendkomposite oder Verblendkunststoff, insbesondere für dentale Zwecke, **dadurch gekennzeichnet,**
- **dass** ein mit dem Verblendwerkstoff zu versehender Grundkörper aus noch nicht dicht gesinterter Oxidkeramik oder Ausgangsstoffen derselben hergestellt wird, auf deren zu verblendender Oberfläche ein Haftvermittler, bestehend aus einem Gemisch aus Feldspat- und Quarzpartikeln im Mischungsverhältnis zwischen 95 : 05 und 10 : 90 sowie aus einem Dispergator, in Form eines Sols aufgetragen wird, wobei dieses Sol in die Oberfläche des noch nicht dicht gesinterten Grundkörpers eindringt,
- **dass** der Grundkörper nach dem Trocknen der zu verblendenden Oberfläche mit dem aufgetragenen Haftvermittler bei Temperaturen von 1250 °C bis 1600 °C dicht gesintert wird, wobei durch dieses Sintern der Oxidkeramik die eingedrungenen Silikatstrukturen fest in die Oxidkeramik eingebunden werden und
- **dass** danach der Verblendwerkstoff auf den nunmehr dicht gesinterten Grundkörper aufgebracht und anschließend bei 850 °C bis 1000 °C gesintert wird oder
- **dass** danach der Verblendwerkstoff auf den nunmehr dicht gesinterten Grundkörper aufgebracht und anschließend gehärtet oder polymerisiert wird.

2. Verfahren nach Anspruch 1, **dadurch gekennzeichnet, dass** das Trocknen bei Umgebungstemperatur oder durch Heizung erfolgt.

3. Haftvermittler in Form eines Sols, eingebracht zwischen einer Oxidkeramik und einem Verblendwerkstoff für dentale Zwecke, welcher aus einem Gemisch aus Feldspat- und Quarzpartikeln im Mischungsverhältnis zwischen 95 : 05 und 10 : 90, sowie aus einem Dispergator in Form von Wasser besteht.

4. Haftvermittler gemäß Anspruch 3, **gekennzeichnet durch** ein Gemisch von Feldspat und Quarz im Mischungsverhältnis zwischen 90 : 10 und 15 : 85.

5. Haftvermittler gemäß Anspruch 3 oder 4, **dadurch gekennzeichnet, dass** die Oxidkeramik Zirkoniumoxid-, Aluminiumoxid- oder Spinell-Keramik und der Verblendwerkstoff Silikatkeramik, Verblendkomposite oder Kunststoff ist.

6. Kit zur Durchführung des Verfahrens gemäß einem oder mehreren der Ansprüche 1 bis 2, bestehend zumindest aus
- wenigstens einem Behältnis, enthaltend einen Verblendwerkstoff,
- wenigstens einem Behältnis mit einem Gemisch aus Feldspat- und Quarzpartikeln im Mischungsverhältnis zwischen 95 :05 und 10 : 90,
- wenigstens einem Behältnis mit einem Dispergator in Form von Wasser,
- Instrumente zum Anmischen des Haftvermittlers und / oder dessen Auftragen sowie
- einer Vorschrift zur Anwendung des Kits.

## Claims

1. Method for using an adhesion promoter for the production of a composite between an oxide ceramic, consisting of one or more metal oxides, and a veneer material to be applied to the oxide ceramics, consisting of silicate ceramic, veneering composites or veneer plastic, in particular for dental purposes, **characterized in**
- **that** a base body to be provided with the veneer material is produced from oxide ceramics which has not yet been densely sintered or starting materials thereof, on the surface of them to be veneered an adhesion promoter, consisting of a mixture of feldspar and quartz particles in a mixing ratio between 95 : 05 and 10 : 90 and of a dispersant, is provided in the form of a sol, wherein this sol penetrates into the surface of the base body not yet densely sintered,
- **that** the base body, after drying of the surface to be veneered, is densely sintered with the applied adhesion promoter at temperatures ranging from 1250°C to 1600°C, wherein the penetrated silicate structures are firmly bound into the oxide ceramic by this sintering of the oxide ceramic, and
- **that** the veneer material is then applied to the now densely sintered base body and is subsequently sintered at a temperature from 850°C to 1000°C or
- **that** the veneer material is then applied to the now densely sintered base body and is subsequently hardened or polymerized.

2. Method according to claim 1, **characterized in that** drying is achieved at ambient temperature or by heating.

3. Adhesion promoter in the form of a sol, introduced between an oxide ceramic and a veneer material for dental purposes, consisting of a mixture of feldspar and quartz particles in a mixing ratio between 95 : 05 and 10 : 90 and of a dispersant in the form of water.

4. Adhesion promoter according to claim 3, **characterized by** a mixture of feldspar and quartz in a mixing ratio between 90 : 10 and 15 : 85.

5. Adhesion promoter according to claim 3 or 4, **characterized in that** the oxide ceramic is zirconium oxide, aluminium oxide or spinel ceramics and the veneer material is silicate ceramics, veneer composites or plastic material.

6. Kit for carrying out the method according to one or more of claims 1 to 2, consisting of not less than
- at least one box containing a veneer material,
- at least one box with a mixture of feldspar and quartz particles in the mixing ratio between 95 : 05 and 10 : 90,
- at least one box containing a dispersant in the form of water,
- instruments for mixing the adhesion promoter and / or for its application, and
- an instruction for using the kit.

## Revendications

1. Procédé pour l'application d'un promoteur d'adhésion afin de fabriquer un composite entre une céramique d'oxyde, se composant d'un ou plusieurs oxydes métalliques, ainsi que d'un matériau de revêtement à appliquer sur la céramique d'oxyde, se composant de céramique à base de silicate, composite d'enrobage ou matière plastique d'enrobage en particulier à usage dentaire, est **caractérisé en ce**
- **qu'**un corps de base destiné à recevoir le matériau de revêtement est formé de céramique d'oxyde par encore frittée de manière étanche ou de ses matières premières et un promoteur d'adhésion, se composant d'un mélange de particules de feldspath et de quartz avec un rapport de mélange allant de 95 : 05 à 10 : 90 ainsi que d'un dispersant sous forme d'un sol, est appliqué sur sa surface à revêtir, et ce sol pénètre dans la surface du corps de base par encore fritté de manière étanche,
- **que** le corps de base est fritté de manière étanche après le séchage de la surface à revêtir avec le promoteur d'adhésion appliqué à des températures de 1250 °C à 1600 °C et les structures de silicate pénétrées sont fermement fixées dans la céramique d'oxyde par son frittage et
- **que** le matériau de revêtement est ensuite appliqué sur le corps de base maintenant fritté de manière étanche et est ensuite fritté à des températures de 850 °C à 1000 °C ou
- **que** le matériau de revêtement est ensuite appliqué sur le corps de base maintenant fritté de manière étanche et est ensuite durci ou polymérisé.

2. Procédé suivant la revendication 1 est **caractérisé en ce que** le séchage s'effectue à température ambiante ou à l'aide du chauffage.

3. Promoteur d'adhésion sous forme d'un sol, introduit entre une céramique d'oxyde et un matériau de revêtement à usage dentaire, qui se compose d'un mélange de particules de feldspath et de quartz avec un rapport de mélange allant de 95 : 05 à 10 : 90 ainsi que d'un dispersant sous forme d'eau.

4. Promoteur d'adhésion suivant la revendication 3 est **caractérisé par** un mélange de feldspath et quartz avec un rapport de mélange allant de 90 : 10 à 15 : 85.

5. Promoteur d'adhésion suivant la revendication 3 ou 4 est **caractérisé en ce que** la céramique d'oxyde est une céramique d'oxyde de zirconium, d'alumine ou de spinelle et le matériau de revêtement est une céramique à base de silicate, un composite d'enrobage ou une matière plastique.

6. Kit pour la réalisation du procédé suivant une ou plusieurs des revendications 1 à 2 se composant
- au moins d'un récipient contenant un matériau de revêtement,
- au moins d'un récipient contenant un mélange de particules de feldspath et de quartz avec un rapport de mélange allant de 95 : 05 à 10 : 90,
- au moins d'un récipient contenant un dispersant sous forme d'eau,
- des instruments pour la production du promoteur d'adhésion et / ou pour son application ainsi que
- des instructions pour l'usage du kit.
